(19) Europäisches Patentamt / European Patent Office / Office européen des brevets

(11) **EP 3 513 735 B1**

(12) **EUROPEAN PATENT SPECIFICATION**

(45) Date of publication and mention
of the grant of the patent:
**25.05.2022 Bulletin 2022/21**

(21) Application number: **17907705.2**

(22) Date of filing: **25.04.2017**

(51) International Patent Classification (IPC):
**A61B 8/08** (2006.01)   **A61B 8/00** (2006.01)
**A61B 8/06** (2006.01)   **G01F 1/66** (2022.01)
**G01P 5/24** (2006.01)   **G01S 7/52** (2006.01)
**G01S 15/58** (2006.01)   **G01S 15/89** (2006.01)

(52) Cooperative Patent Classification (CPC):
**A61B 8/06; A61B 8/00; A61B 8/08; A61B 8/488;**
**G01F 1/663; G01F 1/712; G01S 7/52085;**
**G01S 15/8984; G01S 15/8995;** G01S 15/8979

(86) International application number:
**PCT/KR2017/004373**

(87) International publication number:
**WO 2018/199346 (01.11.2018 Gazette 2018/44)**

(54) **DEVICE AND METHOD FOR GENERATING ULTRASOUND VECTOR DOPPLER IMAGE USING PLANE WAVE SYNTHESIS**

VORRICHTUNG UND VERFAHREN ZUR ERZEUGUNG EINES ULTRASCHALLVEKTORDOPPLERBILDES MIT SYNTHESE EBENER WELLEN

DISPOSITIF ET PROCÉDÉ DESTINÉS À GÉNÉRER UNE IMAGE DOPPLER VECTORIELLE ULTRASONORE À L'AIDE DE LA SYNTHÈSE D'ONDES PLANES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.07.2019 Bulletin 2019/30**

(73) Proprietor: **Sogang University Research Foundation**
**Seoul 04107 (KR)**

(72) Inventors:
• **SONG, Tai-Kyong**
**Seoul 03010 (KR)**
• **YEO, Sunmi**
**Goyang-si**
**Gyeonggi-do 10374 (KR)**
• **YOON, Changhan**
**Busan 46501 (KR)**

(74) Representative: **V.O.**
**P.O. Box 87930**
**2508 DH Den Haag (NL)**

(56) References cited:
JP-A- 2013 141 519     KR-A- 20130 081 626
KR-A- 20150 062 357     KR-B1- 101 652 727
US-A1- 2014 371 594     US-A1- 2015 351 720

• **SOLVEIG SOVIK ALNES ET AL: "Clutter filtering issues in speckle tracking for two-dimensional blood velocity estimation", ULTRASONICS SYMPOSIUM (IUS), 2011 IEEE INTERNATIONAL, IEEE, 18 October 2011 (2011-10-18), pages 427-429, XP032230346, DOI: 10.1109/ULTSYM.2011.0102 ISBN: 978-1-4577-1253-1**
• **FADNES SOLVEIG ET AL: "Robust angle-independent blood velocity estimation based on dual-angle plane wave imaging", IEEE TRANSACTIONS ON ULTRASONICS, FERROELECTRICS AND FREQUENCY CONTROL, IEEE, US, vol. 62, no. 10, 1 October 2015 (2015-10-01), pages 1757-1767, XP011586841, ISSN: 0885-3010, DOI: 10.1109/TUFFC.2015.007108 [retrieved on 2015-10-12]**

**Description**

[Technical Field]

The present invention relates to a technique for acquiring an ultrasound image of a target object through transmission/reception of ultrasound signals, and, more particularly, to an apparatus and method for generating ultrasound vector Doppler images by transmitting/receiving plane waves to/from a target object and combining the received plane waves.

[Background Art]

An ultrasound medical imaging system is an apparatus that transmits/receives ultrasound signals to/from the human body and noninvasively creates images of structures and features inside the human body using information provided by the reflected signals. Such an ultrasound medical imaging apparatus provides various types of clinical information and may employ a Doppler imaging technique to obtain information on movement of a certain moving object to detect the object. Particularly, a Doppler imaging technique used for real-time acquisition of information on a blood flow inside the human body is becoming increasingly important.

Quantitative information on the velocity and change of blood flows inside the human body, one- or two-dimensional distribution of the blood flows, and cross-sectional and three-dimensional shapes of the heart and blood vessel is very important in diagnosing diseases of the heart and circulatory system. Accordingly, techniques for extracting information on the blood flows in various ways have been actively studied. Conventionally, most of the techniques have a problem in that information on a blood flow at a specific location inside the human body cannot be separately observed. Recently, ultrasound Doppler systems have been rapidly technologically advanced to solve this problem and to provide time-dependent dynamic information on blood flow and are thus widely used as an indispensable apparatus for diagnosis of circulatory diseases.

Over the past decades, since existing ultrasonic diagnostic apparatuses have been limited to a role of imaging of *in-vivo* anatomical structures based on B-mode, color flow, Doppler mode, and the like, diagnosis of diseases has required additional functional tests based on anatomical information. In recent years, functional blood flow imaging techniques have been studied extensively. Here, functional blood flow imaging refers to not only imaging of anatomical signals, but also analyzing characteristics of blood flow signals, quantifying the analyzed characteristics, and expressing the quantified analyzed characteristics by indexes. Functional blood flow imaging differs from conventional anatomical imaging in that the functional blood flow imaging creates images of functional aspects of what is happening inside the human body.

A vector Doppler technique has an advantage of providing quantitative information on a moving direction and velocity of a target object and is thus widely used when the traveling direction of a sound wave is perpendicular to an estimated moving direction of a target object, making it difficult to estimate a Doppler phenomenon through a general autocorrelation technique alone. For example, for the carotid artery located parallel to the skin, detection of a blood flow using a general ultrasonic medical imaging device is impossible since an array transducer of the imaging device transmits ultrasound vertically with respect to the skin, causing a Doppler shift frequency of zero. Conversely, the vector Doppler technique is very suitable for diagnosis of vascular diseases in that the blood flow of the carotid artery can be detected without tilting the direction of transmitted ultrasound beams and the velocity and direction of the blood flow can be confirmed at each image point.

In order to achieve proper analysis on characteristics of biological signals, accurate information on the biological signals is required. Here, the information may include blood flow information calculated from a Doppler frequency. Since as many transmission/reception processes as ensembles are required to find the Doppler frequency, a frame rate of a Doppler image is restricted. Here, the frame rate of the Doppler image is a sampling frequency of blood flow information. High-accuracy analysis on the blood flow requires a sufficient amount of blood flow information and thus a sufficiently high sampling rate.

Therefore, there is a need for an image processing technique which can improve sensitivity and accuracy of an ultrasound image while maintaining a frame rate of the ultrasound image at a high level. For this purpose, a plane wave that can have a higher frame rate than a focused beam may be used, and the following prior art document discloses a blood flow imaging method using a plane wave.

<Prior art Document>

Korean Patent Publication No. 2013-0115822 (published on October 22, 2013)
SOLVEIG SOVIK ALNES et al.: "Clutter filtering issues in speckle tracking for two-dimensional blood velocity estimation", ULTRASONICS SYMPOSIUM (IUS), 2011 IEEE INTERNATIONAL, IEEE, 18 October 2011 pages 427-429, may be considered to disclose a method for generating ultrasound Doppler images, comprising the steps of: sequentially and repeatedly transmitting a predetermined number of plane waves having different angles of incidence to an object in a predetermined transmission sequence;receiving reflected signals from the object at different locations generating an ensemble frame by combining, among the received reflected signals, a predetermined number of reflected signals for each of positive and negative angles with respect to a reference direction facing the object; andoutputting a vector Doppler image generated using a plurality of ensemble frames. The said reference also discloses the corresponding apparatus.

[Disclosure]

[Technical Problem]

It is an object of the present invention to provide a vector Doppler technique that can be used when a traveling direction of an ultrasound signal is perpendicular to an estimated moving direction of a target object, wherein the vector Doppler technique can overcome the drawback of a focused beam-based method, i.e., a limited frame rate, while preventing deterioration in image resolution and sensitivity due to use of plane waves.

[Technical Solution]

In accordance with one embodiment of the present invention, the above and other objects of the present invention can be achieved by the independent method claim.

The step of generating the ensemble frame may include: generating one ensemble frame by combining only data values in a region where a predetermined number of reflected signals overlap one another among the received reflected signals, wherein a second ensemble frame temporally subsequent to a first ensemble frame may be generated by reusing a set of reflected signals used in synthesis of the first ensemble frame excluding the first reflected signal in the set, and adding a reflected signal subsequent to the last reflected signal in the set.

The step of generating the ensemble frame may include combining reflected signals corresponding to plane waves having different angles of incidence for each of the positive and negative angles, the reflected signals containing information on a moving direction and velocity of the object.

The step of generating the ensemble frame may include synthesizing the ensemble frame using reflected signals selected from among the reflected signals in a predetermined order, regardless of a transmission/reception sequence.

A frame rate of the vector Doppler image may be a value obtained by dividing a transmission frequency of the plane waves by the number of reflected signals used in synthesis of the ensemble frames and having different angles.

The step of outputting the vector Doppler image may include the steps of: generating an in-phase component and a quadrature component through quadrature demodulation of the ensemble frame; estimating a Doppler shift frequency from the demodulated ensemble frame; and generating a vector Doppler image containing information on a moving direction and velocity of the object, the information being calculated using the estimated Doppler shift frequency.

The step of estimating the Doppler shift frequency may include estimating an average frequency of a power spectrum generated from the in-phase component and the quadrature component and calculating the moving velocity of the object from the estimated average frequency. In addition, the method may further include removing a clutter component from the generated in-phase component and the quadrature component to filter only a Doppler shift component. Further, the step of estimating the Doppler shift frequency may be performed using at least one of autocorrelation using phase shift, cross-correlation using time shift, auto-regression using an all-pole model, and eigen-decomposition using eigenvectors.

In accordance with another aspect of the present invention, the above and other objects of the present invention can be achieved by an apparatus according to the independent apparatus claim.

The processor may generate one ensemble frame by combining only data values in a region where a predetermined number of reflected signals overlap one another among the received reflected signals, wherein a second ensemble frame temporally subsequent to a first ensemble frame may be generated by reusing a set of reflected signals used in synthesis of the first ensemble frame excluding the first reflected signal in the set, and adding a reflected signal subsequent to the last reflected signal in the set.

The processor may generate the ensemble frame by combining reflected signals corresponding to plane waves having different angles of incidence for each of the positive and negative angles, the reflected signals containing information on a moving direction and velocity of the object.

The processor may synthesize the ensemble frame using reflected signals selected from among the reflected signals in a predetermined order, regardless of a transmission/reception sequence.

A frame rate of the vector Doppler image may be a value obtained by dividing a transmission frequency of the plane waves by the number of reflected signals used in synthesis of the ensemble frames and having different angles.

The processor may generate an in-phase component and a quadrature component through quadrature demodulation of the ensemble frame, estimate a Doppler shift frequency from the demodulated ensemble frame, and generate a vector Doppler image containing information on a moving direction and velocity of the object, the information being calculated using the estimated Doppler shift frequency.

The processor may estimate an average frequency of a power spectrum generated from the in-phase component and the quadrature component and calculate the moving velocity of the object from the estimated average frequency. In addition, the processor may further include a clutter filter removing a clutter component from the generated in-phase component and the quadrature component to filter only a Doppler shift component.

The different angles of incidence may be produced through angle-dependent time delay using an array transducer.

Reception of the reflected signals may be achieved by focusing echo signals reflected from the object at different locations using an array transducer.

[Advantageous Effects]

Embodiments of the present invention provide an ultrasound Doppler imaging method which generates an ultrasound vector Doppler image using a plane wave synthesis technique capable of securing a higher frame rate than a focused beam-based technique, wherein the system can improve resolution and sensitivity of the image by synthesizing ensemble data using multi-angle plane waves.

[Description of Drawings]

FIG. 1 is a view illustrating an ultrasound vector Doppler technique.

FIG. 2 is a diagram illustrating a multi-angular vector Doppler method according to embodiments of the present invention.

FIG. 3 is a diagram illustrating a plane wave transmission sequence and synthesis process in the multi-angular vector Doppler method according to embodiments of the present invention.

FIG. 4 is a flowchart illustrating a method for generating ultrasound Doppler images using plane wave synthesis according to one embodiment of the present invention.

FIG. 5 is a view illustrating a vector Doppler method using plane waves.

FIG. 6 is a diagram illustrating a vector Doppler method using multi-angle plane waves.

FIG. 7 is a diagram illustrating a method of generating an ensemble frame in a repeated transmission sequence.

FIG. 8 is a block diagram illustrating an apparatus for generating ultrasound Doppler images using plane wave synthesis according to one embodiment of the present invention.

FIG. 9 and FIG. 10 show an experimental example comparing a vector Doppler image using single-angle plane waves and a corresponding root-mean-square (RMS) error with a vector Doppler image using multi-angle plane waves and a corresponding RMS error.

<List of Reference numerals>

10: pulser
20: transceiver
30: processor
40: display unit

[Best Mode]

A method of generating ultrasound Doppler images according to one embodiment of the present invention comprises the steps of: sequentially and repeatedly transmitting a predetermined number of plane waves having different angles of incidence to an object in a predetermined transmission sequence; receiving reflected signals from the object at different locations; generating an ensemble frame by combining, among the received reflected signals, a predetermined number of reflected signals for each of positive and negative angles with respect to a reference direction facing the object; and outputting a vector Doppler image generated using a plurality of ensemble frames.

[Mode for Invention]

Prior to describing embodiments of the present invention, considerations related to ultrasound techniques suitable for blood flow imaging will be discussed. In addition, after examining problems of typical ultrasound Doppler techniques due to use of plane waves, which is employed by the embodiments of the present invention, technical means employed by the embodiments of the present invention to solve these problems will be sequentially introduced.

It will be described that an ultrasound Doppler technique may be used to detect any moving object and, particularly, a vector Doppler technique is suitable for detecting an object whose estimated moving direction is perpendicular to a traveling direction of an ultrasound signal, such as a blood flow in the carotid artery which is horizontal to the skin.

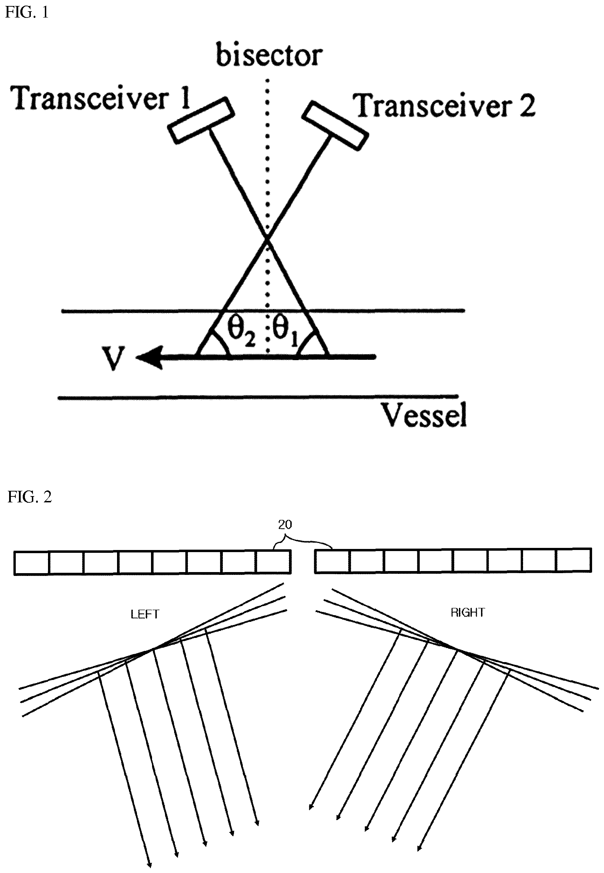

FIG. 1 is a view illustrating an ultrasound vector Doppler technique which provides a calculation method in which Doppler signals are measured at two different locations, followed by combining the Doppler signals based on information on the measurement locations. In this vector Doppler technique, two estimated results are combined based on an assumption that Doppler shift frequencies at any given points as estimated at different locations are reliable. Thus, the vector Doppler technique requires a reliable Doppler shift estimator. In order to estimate movement of an object at a certain point using the Doppler effect, it is necessary to repeatedly acquire sound waves emitted from the object or reflected signals from the object corresponding to pulse waves transmitted from a device to the object. Here, the number of times the reflected signals are repeatedly acquired is referred to as "ensemble", and the reciprocal of a pulse repetition interval is referred to as "pulse repetition frequency (PRF)".

Here, the pulse repetition frequency may be analyzed as a sampling frequency required for estimation of a Doppler shift frequency, and the ensemble is the total number of samples sampled with the pulse repetition frequency at a certain point in time. Accordingly, a Doppler shift frequency measurable according to the Nyquist sampling theorem is restricted up to half the pulse repetition frequency. In addition, if the pulse repetition frequency is excessively low, aliasing occurs in an estimated Doppler shift signal, whereas, if the pulse repetition frequency is excessively high, it is difficult to distinguish the estimated Doppler shift signal from a stationary clutter signal, causing deterioration in Doppler estimation performance.

Since a vector Doppler technique that provides information on the direction and velocity of a blood flow has a limited frame rate in use of typical focused beams, there is a limit to the number of ensemble data. Accordingly, use of focused beams causes deterioration in accuracy of estimation of the velocity and direction of the blood flow and sensitivity of expression of blood flow. In order to solve this problem, use of plane waves has been proposed to increase the frame rate.

A plane wave-based method has a higher frame rate and is thus less limited in the number of ensemble data than a typical focused beam-based method. However, due to lack of transmit-focusing, such a plane wave-based method has problems of deterioration in resolution and sensitivity of an image and inaccurate information on the velocity and direction of a blood flow.

In order to overcome these problems, embodiments of the present invention provide a multi-angular vector Doppler method which can acquire incident frames having various angles through diversification of the plane wave transmission angle to generate one ensemble frame through synthesis of the incident frames, while securing a high frame rate using plane waves.

FIG. 2 is a diagram illustrating a multi-angular vector Doppler method according to embodiments of the present invention, which allows transmission of plane waves at a predetermined tilted angle in right and left directions using an ultrasonic transducer 20, which is a feature of a vector Doppler method, wherein multiple different transmission angles rather than a single uniform transmission angle are realized in each of the right and left directions. Referring to FIG. 2, three steered planes for each of the right and left tilted angles, that is, a total of six steered planes are created and plane waves having different angles of incidence can be transmitted therethrough. Here, the number of plane wave transmission angles may vary depending on the number of received signals used to form an ensemble frame and resolution and sensitivity of an image can be improved with increasing number of plane wave transmission angles. However, since the frame rate is inversely proportional to the number of incident images involved in synthesis of the ensemble frame, the number of plane wave transmission angles needs to be adjusted to an appropriate value depending on usage environments.

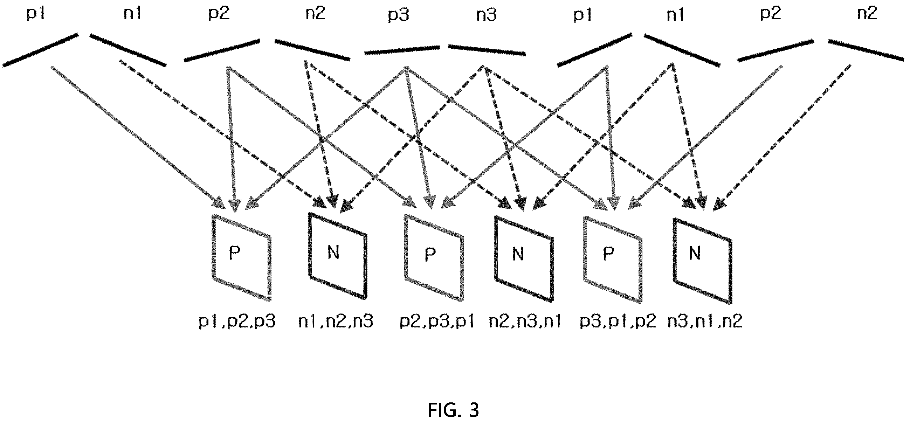

FIG. 3 is a diagram illustrating a plane wave transmission sequence and synthesis process in the multi-angular vector Doppler method according to embodiments of the present invention. Plane waves are generated in predetermined intervals by a pulser and then transmitted to an object by a transducer to generate reflected signals from the object. Here, the plane waves are transmitted at different angles, as shown in FIG. 2, and incident signals may be transmitted in a predetermined transmission sequence, which is a sequentially ordered set of transmission events. As described above, the reciprocal of a pulse repetition interval is a pulse repetition frequency (PRF) and, in FIG. 3, a cycle consisting of p1, n1, p2, n2, p3 and n3 is repeated. Here, an angle formed between an incident signal and the object may be defined in relation to a reference direction facing the object (for example, a direction perpendicular to the object), wherein 'p' denotes a positive direction with respect to the reference direction and 'n' denotes a negative direction with respect to the reference direction. Accordingly, it can be seen that three plane waves having different angles of incidence are transmitted in each of the positive and negative directions.

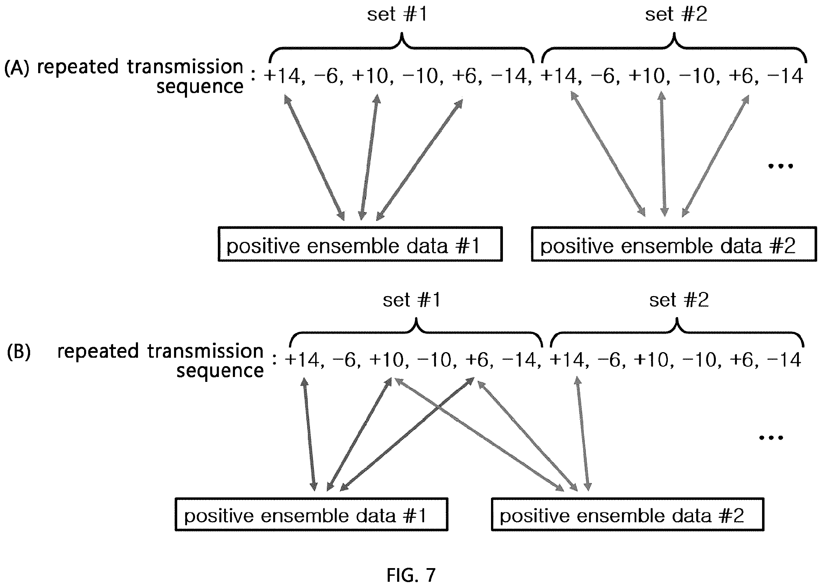

Referring to FIG. 3, in the multi-angular vector Doppler method according to the embodiments of the present invention, reflected signals corresponding to three plane waves having different angles of incidence in each of the positive and negative directions are combined. As described above, ensemble data (a frame) is formed by combining reflected signals, and, herein, three reflected signals are combined into one ensemble data set. Here, in synthesis of the ensemble data from consecutive reflected signals, various reflected signal selection schemes may be employed. In FIG. 3, in order to secure a maximum frame rate, consecutive reflected signals are sequentially slid to form the ensemble data. A difference in frame rate according to selection of reflected signals required for synthesis of the ensemble data will be described further below with reference to FIG. 7.

Although the transmission sequence is illustrated in order of p1, n1, p2, n2, p3, and n3 in FIG. 3, it should be understood that the present invention is not limited thereto and the transmission sequence may be ordered in various ways. For example, the transmission sequence may be in order of n1, p1, n2, p2, n3, and p3, in order of p1, p2, p3, n1, n2, and n3, or in order of n1, n2, n3, p1, p2, and p3.

In order to overcome the drawback of a typical plane wave-based Doppler imaging method, i.e., lower resolution and sensitivity of an image than a focused beam-based imaging method, due to lack of transmission focusing, according to the following embodiments of the present invention, multi-angle plane waves are used to obtain a vector Doppler image. Now, embodiments of the present invention will be described more in detail with reference to the accompanying drawings.

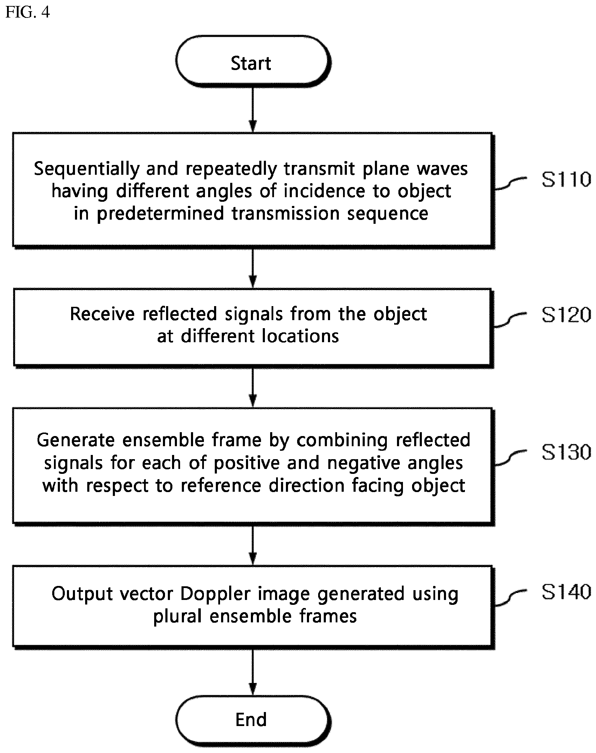

FIG. 4 is a flowchart illustrating a method for generating ultrasound Doppler images using plane wave synthesis according to one embodiment of the present invention, wherein the method includes the following steps:

In step S110, an apparatus for generating ultrasound Doppler images sequentially and repeatedly transmits a predetermined number of plane waves having different angles of incidence to an object in a predetermined transmission sequence. Ultrasound signals generated by a pulser are transmitted in the form of multi-angle plane waves to a target object by a transducer. Multi-angle plane waves may be generated using various technical means, and, for example, through angle-dependent time delay using an array transducer, angle of incidence of a plane wave may be diversified.

In step S120, the apparatus for generating ultrasound Doppler images receives reflected signals from the object at different locations. In a vector Doppler technique, reflected signals in at least two different directions are received to estimate the moving direction and velocity of an object. Reception of reflected signals may be achieved using various technical means, for example, using some elements of the array transducer at different locations or by focusing echo signals reflected from the object at different locations.

In step S130, the apparatus for generating ultrasound Doppler images generates an ensemble frame by combining, among the received reflected signals, a predetermined number of reflected signals for each of positive and negative angles with respect to a reference direction facing the object. In this step, the ensemble frame is generated by combining reflected signals corresponding to plane waves having different angles of incidence for each of the positive and negative angles, wherein the reflected signals contain information on the moving direction and velocity of the object. Accordingly, a frame rate of the vector Doppler image is a value obtained by dividing a transmission frequency of the plane waves by the number of reflected signals used in synthesis of the ensemble frame. For multi-angle plane wave synthesis, since the quality and frame rate of an image are inversely proportional to each other, it is necessary to consider trade-off therebetween in determination of the number of reflected signals used in synthesis of an ensemble frame.

Here, the ensemble frame may be synthesized using reflected signals selected in a predetermined order among the reflected signals, regardless of a transmission/reception sequence. In order words, reflected signals used in synthesis of the ensemble frame may not necessarily be in the order corresponding to the transmitted plane waves, and various frame selection schemes and sequences suitable for usage environments and requirements may be employed.

In step S140, the apparatus for generating ultrasound Doppler images outputs a vector Doppler image generated using a plurality of ensemble frames. For this purpose, an in-phase component and a quadrature component are generated through quadrature demodulation of the ensemble frame; a Doppler shift frequency is estimated from the demodulated ensemble frame; and a vector Doppler image containing information on the moving direction and velocity of the object calculated using the estimated Doppler shift frequency is generated.

Here, the process of estimating the Doppler shift frequency may include estimating an average frequency of a power spectrum generated from the in-phase component and the quadrature component and calculating the moving velocity of the object from the estimated average frequency.

Preferably, step S140 further includes removing a clutter component from the generated in-phase component and the quadrature component to filter only a Doppler shift component. In other words, a clutter signal reflected from a wall of a blood vessel or body tissue rather than signals reflected from a blood flow (or red blood cells) is removed using a clutter filter. Such a clutter signal is a signal that exists in a very low band of a frequency spectrum, and the clutter filter may be designed using an infinite impulse response (IIR) filter. In addition, the clutter filter is useful to reduce error in estimating the Doppler frequency of a blood flow.

Here, estimation of the Doppler shift frequency may be performed using at least one of autocorrelation using phase shift, cross-correlation using time shift, auto-regression using an all-pole model, and eigen-decomposition using eigenvectors. Detailed description of these estimation methods may unnecessarily obscure the subject matter of the present invention and thus will be omitted.

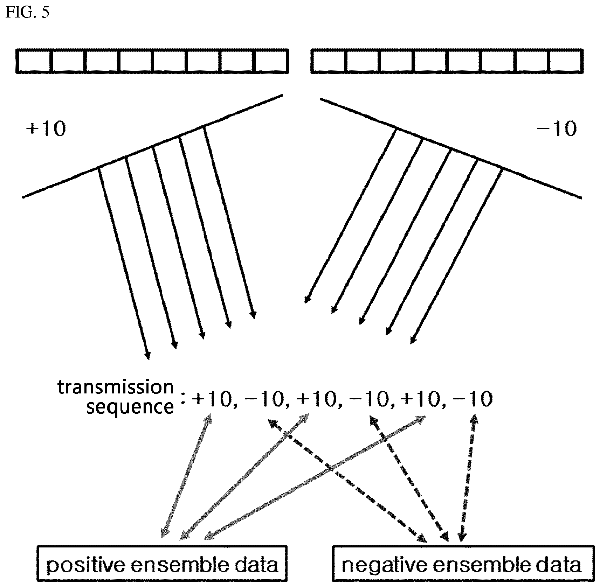

FIG. 5 is a view illustrating a vector Doppler method using plane waves and shows a transmission sequence composed of repetition of a plane wave having a tilted angle of '+10°' in the positive direction and a plane wave having a tilted angle of '-10°' in the negative direction. Here, positive ensemble data is synthesized from reflected signals (indicated by solid lines) corresponding to plane waves incident in the positive direction and a negative ensemble data is synthesized from reflected signals (indicated by dotted lines) corresponding to plane waves incident in the negative direction. For example, when the total pulse repetition frequency (PRF) is 10 kHz as in FIG. 5, a pulse repetition frequency between a first '+10°' data and the next '+10°' data is 5 kHz.

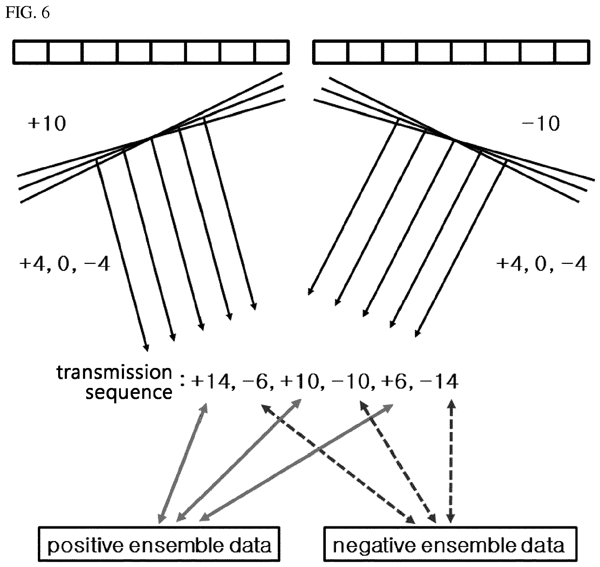

FIG. 6 is a diagram illustrating a vector Doppler method using multi-angle plane waves and shows compounding angles in addition to the tilted angles shown in FIG. 5. Specifically, three compounding angles, '+4°', '0°', and '-4°', are applied to two tilted angles '+10°' and '-10°' to produce a total of six transmission angles, '+14°', '-6°', '+10°', '-10°', '+6°', and '-14°', thereby forming one transmission sequence. Here, positive ensemble data is synthesized from the reflected signals (indicated by solid lines) corresponding to the plane waves incident in the positive direction and negative ensemble data is synthesized from the reflected signals (indicated by dotted lines) corresponding to the plane waves incident in the negative direction.

FIG. 7 is a diagram illustrating a method of generating an ensemble frame in a repeated transmission sequence and shows cases in which ensemble data are synthesized in different ways for a given repeated transmission sequence. For the repeated transmission sequence, '+14°', '-6°', '+10°', '-10°', '+6°', and '-14°' form one set. For convenience of description, only the process of forming positive ensemble data is illustrated, and the same composite operation is used in the process of forming negative ensemble data.

In the first case (A), the positive ensemble data (frame) may be synthesized using only reflected signals corresponding to plane waves having positive angles of incidence in one set of the transmission sequence. In this case, one ensemble data set is generated for each set and there is no reuse of the reflected signals. Referring to FIG. 7, reflected signals constituting 'positive ensemble data #1' are '+14°', '+10°', and '+6°', and reflected signals constituting 'positive ensemble data #2' are also '+14°', '+10°', and '+6°'.

In the second case (B), one positive ensemble data set (frame) may be synthesized using reflected signals corresponding to plane waves having negative angles of incidence in one set of the transmission sequence and the next positive ensemble data (frame) may be synthesized by sequentially reusing the reflected signals constituting the repeated transmission sequence, regardless of whether the reflected signals belong to the same set. In other words, one ensemble frame is generated by combining only data values in a region where a predetermined number of reflected signals (three reflected signals in FIG. 7) among the received reflected signals overlap one another, wherein a second ensemble frame, which is temporally subsequent to a first ensemble frame, is generated by reusing a set of reflected signals used in synthesis of the first ensemble frame, excluding the first reflection signal in the set, and by adding a reflected signal subsequent to the last reflected signal in the set. Referring to FIG. 7, reflected signals constituting 'positive ensemble data #1' are '+14°', '+10°' and '+6°' and reflected signals constituting 'positive ensemble data #2' are '+10°', '+6°', and '+14°', unlike in the first case.

In the first case (A), a pulse repetition frequency (PRF) decreases with increasing number of angles involved in ensemble data synthesis, as represented by Equations 1 and 2.

<Equation 1>

$$N_{Angles} = \frac{PRF_{max}}{PRF_{flow}}$$

wherein $PRF_{max}$ is a reciprocal of $T_{PRF}$, which is a time interval at which an ultrasound signal is transmitted/received to obtain ensemble frame data for one scanning line, and denotes the total pulse repetition frequency; $PRF_{flow}$ denotes a Doppler pulse repetition frequency for estimation of a blood flow velocity; and $N_{Angles}$ denotes the number of angles involved in synthesis.

<Equation 2>

$$\text{Frame rate[Hz]} = \frac{PRF_{flow}}{N_{ensembles}} = \frac{PRF_{max}}{N_{Angles} \times N_{ensembles}}$$

wherein $N_{ensembles}$ denotes the number of ensembles. Referring to Equation 2, in the first case (A), the frame rate decreases with increasing number of reflected signals combined to generate ensemble frames, as well as with increasing number of angles involved in synthesis. In other words, the frame rate changes in inverse proportion to the product of the number of ensembles and the number of angles involved in synthesis.

Conversely, in the second case (B), there is no decrease in pulse repetition frequency in a vector Doppler image after synthesis, as represented by Equations 3 and 4.

<Equation 3>

$$PRF_{flow} = PRF_{max}$$

Since the reflected signals constituting the repeated transmission sequence are sequentially reused in a sliding manner, as shown in FIG. 7, a Doppler pulse repetition frequency for estimation of the blood flow velocity is kept equal to the total pulse repetition frequency.

<Equation 4>

$$\text{Frame rate[Hz]} = \frac{PRF_{flow}}{N_{ensembles}} = \frac{PRF_{max}}{N_{ensembles}}$$

Referring to Equation 4, in the second case (B), the frame rate only decreases with increasing number of reflected signals combined to generate ensemble frames. In other words, the frame rate only changes in inverse proportion to the number of ensembles.

Estimation of the moving velocity of the object by the vector Doppler method using plane waves according to the embodiments of the present invention is expressed as follows:

<Equation 5>

$$V_x = \frac{V_P - V_N}{2 * \sin\theta}, \quad V_z = \frac{V_P + V_N}{2 * (1 + \cos\theta)} \qquad (\theta = 10°)$$

wherein $V_P$ and $V_N$ are estimated from positive (P) data and negative (N) data, respectively, and the transverse velocity and the axial velocity are calculated from $V_P$ and $V_N$, respectively (the tilted angle being given as 10 degrees).

Now, based on Equation 5, the moving velocity and direction of the object may be calculated according to Equation 6.

<Equation 6>

$$V = \sqrt{V_x^2 + V_z^2}, \qquad \emptyset = \tan^{-1}\frac{V_x}{V_z}$$

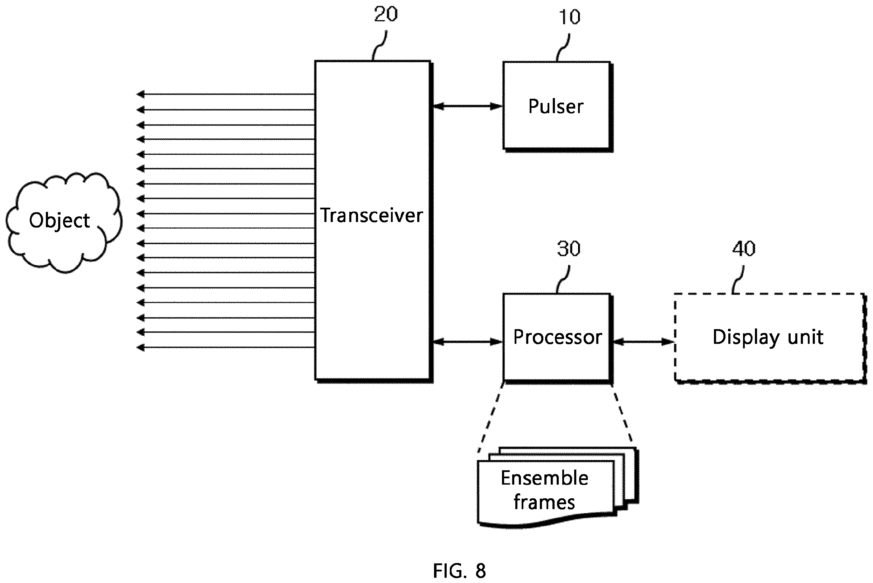

FIG. 8 is a block diagram illustrating an apparatus for generating ultrasound Doppler images using plane wave synthesis according to one embodiment of the present invention, wherein components corresponding to respective processes of the method for generating ultrasound Doppler images shown in FIG. 4 are described again from the viewpoint of hardware configuration. Therefore, only the outline of association between the components will now be described briefly to avoid repeated description.

A pulser 10 generates plane waves at regular intervals and delivers the plane waves to a transceiver 20.

The transceiver 20 may be implemented as an ultrasonic transducer including a single element or plural elements. The transceiver 20 sequentially and repeatedly transmits a predetermined number of plane waves having different angles of incidence to an object in a predetermined transmission sequence and receives reflected signals from the object at different locations.

In addition, the transceiver 20 may produce the different angles of incidence through angle-dependent time delay using an array transducer (not shown). Further, the transceiver 20 may receive the reflected signals by focusing echo signals reflected from the object at different locations using the array transducer (not shown).

A processor 30 generates an ensemble frame by combining, among the received reflected signals, a predetermined number of reflected signals for each of positive and negative angles with respect to a reference direction facing the object and outputs a vector Doppler image generated using a plurality of ensemble frames to a display unit 40.

In order to secure a maximum frame rate, the processor 30 generates one ensemble frame by combining only data values in a region where a predetermined number of reflected signals overlap one another among the received reflected signals, and, preferably, a second ensemble frame temporally subsequent to a first ensemble frame is generated by reusing a set of reflected signals used in synthesis of the first ensemble frame excluding the first reflected signal in the set, and adding a reflected signal subsequent to the last reflected signal in the set.

The processor 30 generates the ensemble frame by combining reflected signals corresponding to plane waves having different angles of incidence angles for each of the positive and negative angles, wherein the reflected signals contain information on the moving direction and velocity of the object. Accordingly, a frame rate of the vector Doppler image may be a value obtained by dividing a transmission frequency of the plane waves by the number of reflected signals used in synthesis of the ensemble frames and having different angles. In addition, the processor 30 may synthesize the ensemble frame using reflected signals selected from among the reflected signals in a predetermined order, regardless of a transmission/reception sequence.

The processor 30 generates an in-phase component and a quadrature component through quadrature demodulation on the ensemble frame, estimates a Doppler shift frequency from the demodulated ensemble frame, and generates a vector Doppler image containing information on the moving direction and velocity of the object, which is calculated using the estimated Doppler shift frequency. Here, the processor 30 may estimate an average frequency of a power spectrum generated from the in-phase component and the quadrature component to calculate the moving velocity of the object from the estimated average frequency. In addition, the processor 30 may further include a clutter filter (not shown) removing a clutter component from the generated in-phase component and the quadrature component to filter only a Doppler shift component.

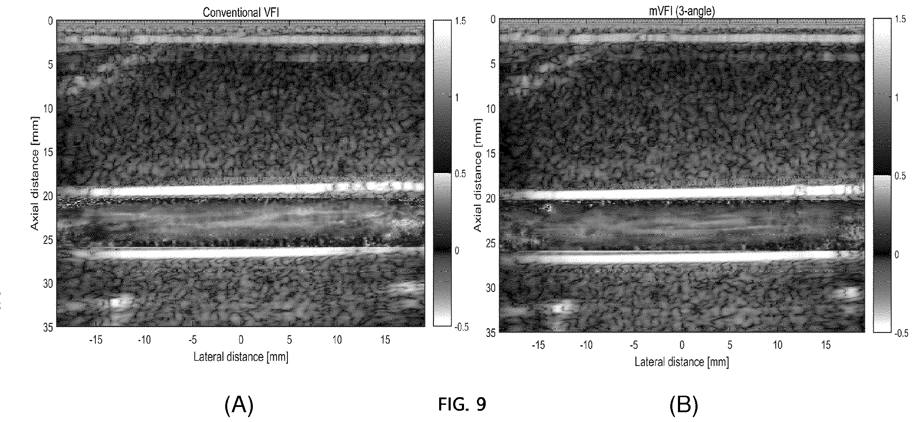

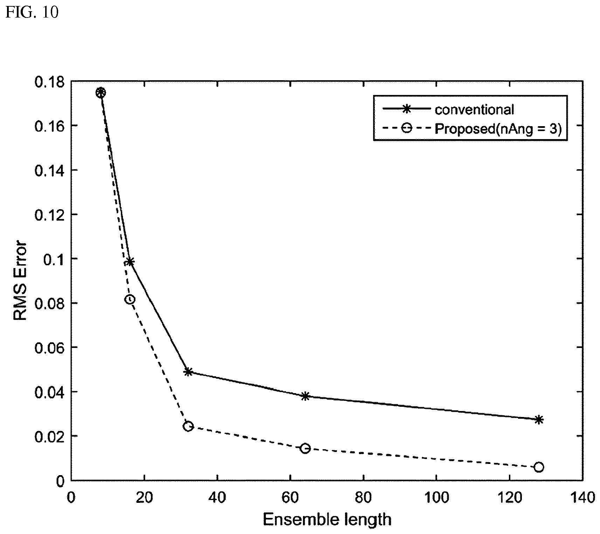

FIG. 9 and FIG. 10 show an experimental example comparing a vector Doppler image using single-angle plane waves and a corresponding root-mean-square (RMS) error with a vector Doppler image using multi-angle plane waves and a corresponding RMS error.

Referring to FIG. 9, it can be seen that a vector Doppler image (B) using multi-angle plane waves according to the embodiments of the present invention exhibits higher Doppler sensitivity than a conventional vector Doppler image (A) using single-angle plane waves. In addition, referring to FIG. 10, it can be seen that a vector Doppler method using multi-angle plane waves can measure a flow rate more accurately and thus can further improve the sensitivity and accuracy of a Doppler image than a vector Doppler method using single-angle plane waves.

Embodiments of the present invention may also be embodied as computer readable code on a computer-readable medium. Here, the computer-readable medium may include any data storage device that can store data readable by a computer system.

Examples of the computer-readable medium may include ROMs, RAMs, CD-ROMs, magnetic tapes, floppy disks, and optical data storage devices. In addition, the computer-readable medium may also be distributed over networked computer systems such that the computer-readable code can be stored and executed in a distributed fashion. Also, functional programs, code, and code segments for accomplishing the present invention may be easily construed as within the scope of the invention by programmers skilled in the art to which the present invention pertains.

Although some embodiments have been described herein, it should be understood by those skilled in the art that these embodiments are given by way of illustration only and the present invention is not limited thereto. In addition, it should be understood that various modifications, variations, and alterations can be made by those skilled in the art without departing from the scope of the present invention. Therefore, the scope of the invention should be limited only by the accompanying 2. claims.

[Industrial Applicability]

The embodiments of the present invention provide an ultrasound Doppler imaging method which generates an ultrasound vector Doppler image using a plane wave synthesis technique capable of securing a higher frame rate than a focused beam-based technique, wherein the system can improve resolution and sensitivity of the image by synthesizing ensemble data using multi-angle plane waves. Particularly, in synthesis of the ensemble data, a new ensemble frame is synthesized by sequentially reusing a set of reflected signals used in synthesis of a previous ensemble frame excluding the first reflected signal in the set, and adding a reflected signal subsequent to the last reflected signal in the set, thereby effectively suppressing reduction in frame rate.

**Claims**

1.    A method for generating ultrasound Doppler images, comprising the steps of:

   sequentially and repeatedly transmitting a predetermined number of plane waves having different angles of incidence to an object in a predetermined transmission sequence;
   receiving reflected signals from the object at different locations;
   generating an ensemble frame by combining, among the received reflected signals, a predetermined number of reflected signals for each of positive and negative angles with respect to a reference direction facing the object, wherein one ensemble frame is generated by combining only data values in a region where a predetermined number of reflected signals overlap one another among the received reflected signals; and
   outputting a vector Doppler image generated using a plurality of ensemble frames.

2.    The method according to claim 1, wherein the step of generating the ensemble frame comprises:
   generating a second ensemble frame temporally subsequent to a first ensemble frame by reusing a set of reflected signals used in synthesis of the first ensemble frame excluding the first reflected signal in the set, and adding a reflected signal subsequent to the last reflected signal in the set.

3.    The method according to claim 1, wherein the step of generating the ensemble frame comprises:
   combining reflected signals corresponding to plane waves having different angles of incidence for each of the positive and negative angles, the reflected signals containing information on a moving direction and velocity of the object.

4.    The method according to claim 1, wherein the step of generating the ensemble frame comprises:
   synthesizing the ensemble frame using reflected signals selected from among the reflected signals in a predetermined order, regardless of a transmission/reception sequence.

5.    The method according to claim 1, wherein a frame rate of the vector Doppler image is a value obtained by dividing a transmission frequency of the plane waves by the number of reflected signals used in synthesis of the ensemble frames and having different angles.

6.    The method according to claim 1, wherein the step of outputting the vector Doppler image comprises the steps of:

   generating an in-phase component and a quadrature component through quadrature demodulation of the ensemble frame;
   estimating a Doppler shift frequency from the demodulated ensemble frame; and
   generating a vector Doppler image containing information on a moving direction and velocity of the object, the information being calculated using the estimated Doppler shift frequency.

7.    The method according to claim 6, wherein the step of estimating the Doppler shift frequency comprises:
   estimating an average frequency of a power spectrum generated from the in-phase component and the quadrature component and calculating the moving velocity of the object from the estimated average frequency.

**8.** The method according to claim 6, further comprising:
removing a clutter component from the generated in-phase component and the quadrature component to filter only a Doppler shift component.

**9.** The method according to claim 6, wherein the step of estimating the Doppler shift frequency is performed using at least one of autocorrelation using phase shift, cross-correlation using time shift, auto-regression using an all-pole model, and eigen-decomposition using eigenvectors.

**10.** An apparatus for generating ultrasound Doppler images, comprising:

a pulser generating plane waves at regular intervals;
a transceiver sequentially and repeatedly transmitting a predetermined number of plane waves having different angles of incidence to an object in a predetermined transmission sequence and receiving reflected signals from the object at different locations; and
a processor generating an ensemble frame by combining, among the received reflected signals, a predetermined number of reflected signals for each of positive and negative angles with respect to a reference direction facing the object, wherein one ensemble frame is generated by combining only data values in a region where a predetermined number of reflected signals overlap one another among the received reflected signals, and outputting a vector Doppler image generated using a plurality of ensemble frames.

**11.** The apparatus according to claim 10, wherein the processor generates a second ensemble frame temporally subsequent to a first ensemble frame by reusing a set of reflected signals used in synthesis of the first ensemble frame excluding the first reflected signal in the set, and adding a reflected signal subsequent to the last reflected signal in the set.

**12.** The ultrasound Doppler image generator according to claim 10, wherein the processor generates the ensemble frame by combining reflected signals corresponding to plane waves having different angles of incidence angles for each of the positive and negative angles, the reflected signals containing information on a moving direction and velocity of the object.

**13.** The ultrasound Doppler image generator according to claim 10, wherein the processor synthesizes the ensemble frame using reflected signals selected from among the reflected signals in a predetermined order, regardless of a transmission/reception sequence.

**14.** The ultrasound Doppler image generator according to claim 10, wherein a frame rate of the vector Doppler image is a value obtained by dividing a transmission frequency of the plane waves by the number of reflected signals used in synthesis of the ensemble frames and having different angles.

**15.** The apparatus according to claim 10, wherein the processor generates an in-phase component and a quadrature component through quadrature demodulation of the ensemble frame, estimates a Doppler shift frequency from the demodulated ensemble frame, and generates a vector Doppler image containing information on a moving direction and velocity of the object, the information being calculated using the estimated Doppler shift frequency.

**Patentansprüche**

**1.** Verfahren zum Erzeugen von Ultraschall-Doppler-Bildern, das die Schritte umfasst:

sequentielle und wiederholte Übertragung einer vorbestimmten Anzahl von ebenen Wellen mit unterschiedlichen Einfallswinkeln auf ein Objekt in einer vorbestimmten Übertragungssequenz;
Empfangen der vom Objekt reflektierten Signale an verschiedenen Stellen;
Erzeugen eines Gesamtframes durch Kombinieren einer vorbestimmten Anzahl von reflektierten Signalen unter den empfangenen reflektierten Signalen für die jeweils positiven und negativen Winkel in Bezug auf eine Referenzrichtung, die dem Objekt zugewandt ist, wobei ein Gesamtframe durch Kombinieren nur von Datenwerten in einem Bereich erzeugt wird, in dem eine vorbestimmte Anzahl von reflektierten Signalen einander unter den empfangenen reflektierten Signalen überlappen; und
Ausgeben eines Vektor-Doppler-Bildes, das unter Verwendung mehrerer Gesamtframes erzeugt wurde.

2. Verfahren nach Anspruch 1, wobei der Schritt des Erzeugens des Gesamtframes umfasst:
Erzeugen eines zweiten Gesamtframes, der zeitlich auf einen ersten Gesamtframe folgt, durch Wiederverwenden eines Satzes von reflektierten Signalen, die bei der Synthese des ersten Gesamtframes verwendet wurden, mit Ausnahme des ersten reflektierten Signals in dem Satz, und Hinzufügen eines reflektierten Signals, das auf das letzte reflektierte Signal in dem Satz folgt.

3. Verfahren nach Anspruch 1, wobei der Schritt des Erzeugens des Gesamtframes umfasst:
Kombinieren von reflektierten Signalen, die ebenen Wellen mit unterschiedlichen Einfallswinkeln für die jeweils positiven und negativen Winkel entsprechen, wobei die reflektierten Signale Informationen über eine Bewegungsrichtung und -geschwindigkeit des Objekts enthalten.

4. Verfahren nach Anspruch 1, wobei der Schritt des Erzeugens des Gesamtframes umfasst:
Synthetisieren des Gesamtframes unter Verwendung von reflektierten Signalen, die aus den reflektierten Signalen in einer vorbestimmten Reihenfolge ausgewählt werden, unabhängig von einer Übertragungs-/Empfangssequenz.

5. Verfahren nach Anspruch 1, wobei die Framerate des Vektor-Doppler-Bildes ein Wert ist, der durch das Teilen einer Übertragungsfrequenz der ebenen Wellen durch die Anzahl der reflektierten Signale erhalten wird, die bei der Synthese der Gesamtframes verwendet werden und unterschiedliche Winkel aufweisen.

6. Verfahren nach Anspruch 1, wobei der Schritt des Ausgebens des Vektor-Doppler-Bildes die Schritte umfasst:

Erzeugen einer In-Phase-Komponente und einer Quadratur-Komponente durch Quadratur-Demodulation des Gesamtframes;
Schätzen einer Doppler-Verschiebungsfrequenz aus dem demodulierten Gesamtframe und
Erzeugen eines Vektor-Doppler-Bildes, das Informationen über die Bewegungsrichtung und -geschwindigkeit des Objekts enthält, wobei die Informationen unter Verwendung der geschätzten Doppler-Verschiebungsfrequenz berechnet werden.

7. Verfahren nach Anspruch 6, wobei der Schritt des Schätzens der Doppler-Verschiebungsfrequenz umfasst:
Schätzen einer Durchschnittsfrequenz eines aus der In-Phase-Komponente und der Quadratur-Komponente erzeugten Leistungsspektrums und Berechnen der Bewegungsgeschwindigkeit des Objekts aus der geschätzten Durchschnittsfrequenz.

8. Verfahren nach Anspruch 6, ferner umfassend:
Entfernen einer Clutter-Komponente aus der erzeugten In-Phase-Komponente und der Quadratur-Komponente, um nur eine Doppler-Verschiebungskomponente zu filtern.

9. Verfahren nach Anspruch 6, wobei der Schritt des Schätzens der Doppler-Verschiebungsfrequenz unter Verwendung von wenigstens der Autokorrelation unter Verwendung der Phasenverschiebung und/oder Kreuzkorrelation unter Verwendung der Zeitverschiebung und/oder Autoregression unter Verwendung eines Allpolmodells und/oder Eigenzerlegung unter Verwendung von Eigenvektoren durchgeführt wird.

10. Einrichtung zum Erzeugen von Ultraschall-Doppler-Bildern, die umfasst:

einem Impulsgeber, der in regelmäßigen Abständen ebene Wellen erzeugt;
einen Sender-Empfänger, der sequentiell und wiederholt eine vorbestimmte Anzahl von ebenen Wellen mit verschiedenen Einfallswinkeln zu einem Objekt in einer vorbestimmten Übertragungssequenz überträgt und reflektierte Signale von dem Objekt an verschiedenen Stellen empfängt; und
einen Prozessor, der einen Gesamtframe durch Kombinieren einer vorbestimmten Anzahl von reflektierten Signalen unter den empfangenen reflektierten Signalen für die jeweils positiven und negativen Winkel in Bezug auf eine Referenzrichtung, die dem Objekt zugewandt ist, erzeugt, wobei ein Gesamtframe durch Kombinieren nur von Datenwerten in einem Bereich, in dem eine vorbestimmte Anzahl von reflektierten Signalen einander unter den empfangenen reflektierten Signalen überlappen, und Ausgeben eines Vektor-Doppler-Bildes, das unter Verwendung mehrerer Gesamtframes erzeugt wurde, erzeugt wird.

11. Einrichtung nach Anspruch 10, wobei der Prozessor einen zweiten Gesamtframe erzeugt, der zeitlich auf einen ersten Gesamtframe folgt, durch Wiederverwenden eines Satzes von reflektierten Signalen, die bei der Synthese des ersten Gesamtframes verwendet wurden, mit Ausnahme des ersten reflektierten Signals in dem Satz, und Hinzufügen eines reflektierten Signals, das auf das letzte reflektierte Signal in dem Satz folgt.

12. Ultraschall-Doppler-Bildgenerator nach Anspruch 10, wobei der Prozessor den Gesamtframe durch Kombinieren reflektierter Signale erzeugt, die ebenen Wellen mit unterschiedlichen Einfallswinkeln für die jeweils positiven und negativen Winkel entsprechen, wobei die reflektierten Signale Informationen über eine Bewegungsrichtung und -geschwindigkeit des Objekts enthalten.

13. Ultraschall-Doppler-Bildgenerator nach Anspruch 10, wobei der Prozessor den Gesamtframe unter Verwendung reflektierter Signale synthetisiert, die aus den reflektierten Signalen in einer vorbestimmten Reihenfolge ausgewählt werden, unabhängig von einer Übertragungs-/Empfangssequenz.

14. Ultraschall-Doppler-Bildgenerator nach Anspruch 10, wobei die Framerate des Vektor-Doppler-Bildes ein Wert ist, der durch das Teilen einer Übertragungsfrequenz der ebenen Wellen durch die Anzahl der reflektierten Signale erhalten wird, die bei der Synthese der Gesamtframes verwendet werden und unterschiedliche Winkel aufweisen.

15. Einrichtung nach Anspruch 10, wobei der Prozessor eine In-Phase-Komponente und eine Quadratur-Komponente durch Quadratur-Demodulation des Gesamtframes erzeugt, eine Doppler-Verschiebungsfrequenz aus dem demodulierten Gesamtframe schätzt und ein Vektor-Doppler-Bild erzeugt, das Informationen über eine Bewegungsrichtung und -geschwindigkeit des Objekts enthält, wobei die Informationen unter Verwendung der geschätzten Doppler-Verschiebungsfrequenz berechnet werden.

**Revendications**

1. Procédé de génération d'images Doppler à ultrasons, comprenant les étapes consistant à :

transmettre séquentiellement et de manière répétée un nombre prédéterminé d'ondes planes ayant différents angles d'incidence sur un objet dans une séquence de transmission prédéterminée ;
recevoir des signaux réfléchis de l'objet à différents emplacements ;
générer une trame d'ensemble en combinant, parmi les signaux réfléchis reçus, un nombre prédéterminé de signaux réfléchis pour chacun des angles positifs et négatifs par rapport à une direction de référence faisant face à l'objet, une trame d'ensemble étant générée en combinant uniquement des valeurs de données dans une région où un nombre prédéterminé de signaux réfléchis se chevauchent parmi les signaux réfléchis reçus ; et
délivrer une image Doppler vectorielle générée à l'aide d'une pluralité de trames d'ensemble.

2. Procédé selon la revendication 1, dans lequel l'étape de génération de la trame d'ensemble comprend l'étape consistant à :
générer une deuxième trame d'ensemble postérieurement dans le temps à une première trame d'ensemble en réutilisant un ensemble de signaux réfléchis utilisés dans la synthèse de la première trame d'ensemble à l'exclusion du premier signal réfléchi dans l'ensemble, et en ajoutant un signal réfléchi à la suite du dernier signal réfléchi dans l'ensemble .

3. Procédé selon la revendication 1, dans lequel l'étape de génération de la trame d'ensemble comprend l'étape consistant à :
combiner des signaux réfléchis correspondant à des ondes planes ayant des angles d'incidence différents pour chacun des angles positifs et négatifs, les signaux réfléchis contenant des informations sur une direction de déplacement et une vitesse de l'objet.

4. Procédé selon la revendication 1, dans lequel l'étape de génération de la trame d'ensemble comprend l'étape consistant à :
synthétiser la trame d'ensemble à l'aide de signaux réfléchis sélectionnés parmi les signaux réfléchis dans un ordre prédéterminé, indépendamment d'une séquence d'émission/réception.

5. Procédé selon la revendication 1, dans lequel une cadence de trame de l'image vectorielle Doppler est une valeur obtenue en divisant une fréquence de transmission des ondes planes par le nombre de signaux réfléchis utilisés dans la synthèse des trames d'ensemble et ayant des angles différents.

6. Procédé selon la revendication 1, dans lequel l'étape de délivrance de l'image Doppler comprend les étapes consistant à :

générer une composante en phase et une composante en quadrature par démodulation en quadrature de la trame d'ensemble ;
estimer une fréquence de décalage Doppler à partir de la trame d'ensemble démodulée ; et
générer une image Doppler vectorielle contenant des informations sur une direction de déplacement et une vitesse de l'objet, les informations étant calculées en utilisant la fréquence de décalage Doppler estimée.

7.  Procédé selon la revendication 6, dans lequel l'étape d'estimation de la fréquence de décalage Doppler comprend l'étape consistant à :
estimer une fréquence moyenne d'un spectre de puissance généré à partir de la composante en phase et de la composante en quadrature et calculer la vitesse de déplacement de l'objet à partir de la fréquence moyenne estimée.

8.  Procédé selon la revendication 6, comprenant en outre l'étape consistant à :
supprimer une composante de fouillis de la composante en phase générée et de la composante en quadrature pour filtrer uniquement une composante de décalage Doppler.

9.  Procédé selon la revendication 6, dans lequel l'étape d'estimation de la fréquence de décalage Doppler est effectuée en utilisant au moins l'une parmi l'autocorrélation utilisant le décalage de phase, l'intercorrélation utilisant le décalage temporel, l'auto-régression utilisant un modèle tous pôles, et la décomposition propre utilisant des vecteurs propres.

10.  Appareil pour générer des images Doppler à ultrasons, comprenant :

un pulseur générant des ondes planes à intervalles réguliers ;
un émetteur-récepteur transmettant séquentiellement et de manière répétée un nombre prédéterminé d'ondes planes ayant différents angles d'incidence sur un objet dans une séquence de transmission prédéterminée et recevant des signaux réfléchis depuis l'objet à différents emplacements ; et un processeur générant une trame d'ensemble en combinant, parmi les signaux réfléchis reçus, un nombre prédéterminé de signaux réfléchis pour chacun des angles positifs et négatifs par rapport à une direction de référence faisant face à l'objet, une trame d'ensemble étant générée en combinant uniquement des valeurs de données dans une région où un nombre prédéterminé de signaux réfléchis se chevauchent parmi les signaux réfléchis reçus, et délivrer une image Doppler vectorielle générée à l'aide d'une pluralité de trames d'ensemble.

11.  Appareil selon la revendication 10, dans lequel le processeur génère une deuxième trame d'ensemble postérieurement dans le temps à une première trame d'ensemble en réutilisant un ensemble de signaux réfléchis utilisés dans la synthèse de la première trame d'ensemble à l'exclusion du premier signal réfléchi dans l'ensemble, et en ajoutant un signal réfléchi suivant le dernier signal réfléchi dans l'ensemble.

12.  Générateur d'images Doppler à ultrasons selon la revendication 10, dans lequel le processeur génère la trame d'ensemble en combinant des signaux réfléchis correspondant à des ondes planes ayant différents angles d'incidence pour chacun des angles positifs et négatifs, les signaux réfléchis contenant des informations sur une direction de déplacement et une vitesse de l'objet.

13.  Générateur d'images Doppler à ultrasons selon la revendication 10, dans lequel le processeur synthétise la trame d'ensemble en utilisant des signaux réfléchis sélectionnés parmi les signaux réfléchis dans un ordre prédéterminé, indépendamment d'une séquence d'émission/réception.

14.  Générateur d'images Doppler à ultrasons selon la revendication 10, dans lequel une cadence de trame de l'image Doppler vectorielle est une valeur obtenue en divisant une fréquence de transmission des ondes planes par le nombre de signaux réfléchis utilisés dans la synthèse des trames d'ensemble et ayant des angles différents.

15.  Appareil selon la revendication 10, dans lequel le processeur génère une composante en phase et une composante en quadrature par démodulation en quadrature de la trame d'ensemble, estime une fréquence de décalage Doppler à partir de la trame d'ensemble démodulée, et génère une image Doppler vectorielle contenant des informations sur une direction de déplacement et une vitesse de l'objet, l'information étant calculée à l'aide de la fréquence de décalage Doppler estimée.

FIG. 1

FIG. 2

FIG. 3

FIG. 4

```
                    ┌──────────────┐
                    │    Start     │
                    └──────┬───────┘
                           │
                           ▼
┌────────────────────────────────────────────────┐
│  Sequentially and repeatedly transmit plane waves │        S110
│   having different angles of incidence to object  │
│      in predetermined transmission sequence       │
└────────────────────────┬───────────────────────┘
                           │
                           ▼
┌────────────────────────────────────────────────┐
│      Receive reflected signals from the object   │        S120
│              at different locations              │
└────────────────────────┬───────────────────────┘
                           │
                           ▼
┌────────────────────────────────────────────────┐
│  Generate ensemble frame by combining reflected  │        S130
│   signals for each of positive and negative angles│
│  with respect to reference direction facing object│
└────────────────────────┬───────────────────────┘
                           │
                           ▼
┌────────────────────────────────────────────────┐
│   Output vector Doppler image generated using    │        S140
│              plural ensemble frames              │
└────────────────────────┬───────────────────────┘
                           │
                           ▼
                    ┌──────────────┐
                    │     End      │
                    └──────────────┘
```

FIG. 5

transmission
sequence : +10, −10, +10, −10, +10, −10

| positive ensemble data | negative ensemble data |

FIG. 6

+10

−10

+4, 0, −4

+4, 0, −4

transmission sequence : +14, −6, +10, −10, +6, −14

| positive ensemble data | negative ensemble data |

set #1            set #2

(A) repeated transmission
sequence : +14, −6, +10, −10, +6, −14, +14, −6, +10, −10, +6, −14

• • •

positive ensemble data #1     positive ensemble data #2

set #1            set #2

(B) repeated transmission
sequence : +14, −6, +10, −10, +6, −14, +14, −6, +10, −10, +6, −14

• • •

positive ensemble data #1     positive ensemble data #2

FIG. 7

FIG. 8

Conventional VFI

mVFI (3-angle)

(A)                    FIG. 9                    (B)

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20130115822

### Non-patent literature cited in the description

- Clutter filtering issues in speckle tracking for two-dimensional blood velocity estimation. **SOLVEIG SOVIK ALNES et al.** ULTRASONICS SYMPOSIUM (IUS). IEEE INTERNATIONAL, 18 October 2011, 427-429